# FASCICULE DE BREVET EUROPEEN

(11) **EP 0 678 026 B1**
(45) Date de publication et mention de la délivrance du brevet: **28.08.1996**
(21) Numéro de dépôt: 94903936.6
(22) Date de dépôt: 03.01.1994
(51) Int. Cl.: A61K 31/55

(54) **APPLICATION DE LA CARBAMAZEPINE ET DE L'OXCARBAZEPINE DANS LE TRAITEMENT DE LA MALADIE DE PARKINSON ET DES SYNDROMES PARKINSONIENS**
VERWENDUNG VON CARBAMAZEPINE UND OXCARBAZEPINE ZUR BEHANDLUNG VON PARKINSON UND DEN PARKINSON-SYNDROMEN
APPLICATION OF CARBAMAZEPINE AND OXCARBAZEPINE IN THE TREATMENT OF PARKINSON'S DISEASE AND PARKINSONIAN SYNDROMES

(30) Priorité: 07.01.1993 FR 9300074
(43) Date de publication de la demande: 25.10.1995
(73) Titulaire: RHONE-POULENC RORER S.A., 92160 Antony (FR)
(72) Inventeur: BOIREAU, Alain, F-94370 Sucy-en-Brie (FR); DOBLE, Adam, F-75005 Paris (FR); DUBEDAT, Pierre, F-94130 Nogent-sur-Marne (FR); LOUVEL, Erik, F-75010 Paris (FR); MEUNIER, Mireille, F-91410 Dourdan (FR); MIQUET, Jean-Marie, F-91400 Orsay (FR); STUTZMANN, Jean-Marie, F-94440 Villecresnes (FR); BORDIER, Françoise, F-75020 Paris (FR)
(74) Mandataire: Savina, Jacques
(86) Numéro de dépôt international: FR9400004
(87) Numéro de publication internationale: WO9415610

(56) Documents cités:
- EP-A- 0 050 589
- EP-A- 0 517 347
- LIFE SCIENCES vol. 54, no. 4 , 1994 pages 245 - 252 STACEY A ET AL. 'THE NOVEL ANTICONVULSANT LAMOTRIGINE PREVENTS DOPAMINE DEPLETION IN C57 BLACK MICE IN THE MPTP ANIMAL MODEL OF PARKINSON'S DISEASE'
- CHEMICAL ABSTRACTS, vol. 115, no. 15, 14 Octobre 1991, Columbus, Ohio, US; abstract no. 150162m, FUKUDA ET AL. 'PROTECTIVE EFFECTS OF ANTICONVULSANT DRUGS AGAINST DELAYED NEURONAL DEATH IN GERBIL HIPPOCAMPUS'
- NEUROREPORT vol. 1, no. 1 , 1990 page 26-28 H. LAMPE ET AL. 'CARBAMAZEPINE BLOCKS NMDA-ACTIVATED CURRENTS IN CULTURED SPINAL CORD NEURONS'
- EXPERIENTIA vol. 48, no. 8 , Aoüt 1992 pages 751 - 753 J.M. LANCASTER ET AL. 'CARBAMAZEPINE INHIBITS NMDA-INDUCED DEPOLARIZATIONS IN CORTICAL WEDGES PREPARED FROM DBA/2 MICE'
- SOC. NEUROSCI. ABSTR. vol. 18, no. 1-2 , 1992 page 381 T. DALKARA ET AL. 'CARBAMAZEPINE AND PHENYTOIN INHIBIT NMDA RECEPTOR-MEDIATED CURRENTS IN THE RAT HIPPOCAMPUS'
- TRENDS IN NEUROSCIENCES vol. 12, no. 8 , 1989 pages 285 - 286 T. KLOCKGETHER 'EXCITATORY AMINO ACIDS AND THE BASAL GANGLIA: IMPLICATIONS FOR THE THERAPY OF PARKINSON'S DISEASE'
- NEUROLOGY vol. 37, no. SUPL , 1987 page 339 E. MELAMED ET AL. 'EFFECT OF ANTICONVULSANTS ON THE NEUROTOXICITY OF MPTP AND STRIATAL DOPAMINE METABOLISM IN MICE'
- J.E.F. REYNOLDS 'MARTINDALE, THE EXTRA PHARMACOPOEIA' 1989 , THE PHARMACEUTICAL PRESS , LONDON
- J.E.F. REYNOLDS: 'MARTINDALE, THE EXTRA PHARMACOPOEIA', 1989, THE PHARMACEUTICAL PRESS, LONDON, GB

## Description

La présente invention concerne une nouvelle application thérapeutique des anticonvulsivants choisis parmi la carbamazépine et l'oxcarbazépine ou les sels pharmaceutiquement acceptables de ces composés.

La carbamazépine et l'oxcarbazépine sont décrits comme anticonvulsivants et antiépileptiques notamment dans le brevet EP 50589.

Il a maintenant été trouvé de manière surprenante que ces composés peuvent aussi être utilisés dans le traitement de la maladie de Parkinson et des syndromes parkinsoniens.

Il est connu que la neurotoxine MPTP (1-méthyl-4-phényl-1,2,3,6-tétrahydropyridine) induit un syndrome similaire à la maladie de Parkinson. Ce syndrome résulte d'une dégénération des neurones nigrostriataux dopaminergiques chez les primates (R. S. BURNS et coll., Proc. Natl. Acad. Sci., 80, 4546-4550 (1983), chez l'homme (J. W. LANGSTON et coll., Science, 219, 979-980 (1983)) et chez la souris (R. E. HEIKKILA et coll., Science, 224, 1451-1453 (1984).

L'activité des produits a donc été mise en évidence chez la souris en mesurant les diminutions des taux de dopamine striatale, d'acide dihydroxy-3,4 phénylacétique et d'acide homovanillique induites par la MPTP comparées à celles des animaux témoins.

On injecte, 3 fois à 2 heures d'intervalle, 15 mg/kg de MPTP par voie intrapéritonéale à des souris (C57BL/6) pesant 20-25 g. Trente minutes avant la première injection de MPTP, puis 2 heures et 30 minutes, 5 heures et 30 minutes et 7 heures et 30 minutes après la première injection de MPTP on administre selon le produit de 1 à 40 mg/kg du produit à étudier. Durant les 3 jours suivants, on administre 2 fois par jour selon le produit de 1 à 40 mg/kg du produit à étudier. Les souris sont sacrifiées 8 jours après l'injection de MPTP. Le striatum est disséqué et conservé à -70°C jusqu'au moment de son analyse. Les taux de dopamine, d'acide dihydroxy-3,4 phénylacétique et d'acide homovanillique sont mesurés par chromatographie liquide haute pression avec une détection électrochimique. Les analyses statistiques sont effectuées en utilisant ANOVA suivi par le test de SCHEFFE.

Les résultats obtenus avec des doses de 20 mg/kg de carbamazépine sont mentionnés dans le tableau suivant :

| | taux d'acide dihydroxy-3,4 phénylacétique pmol/mg dans le striatum (% par rapport aux témoins) | taux d'acide homovanillique pmol/mg dans le striatum (% par rapport aux témoins) | taux de dopamine pmol/mg dans le striatum (% par rapport aux témoins) |
|---|---|---|---|
| témoins | 54±3 | 95±3 | 856±27 |
| animaux ne recevant que de la MPTP | 22±4 (-59%) | 62±3 (-35%) | 415±22 (-52%) |
| animaux traités avec la carbamazépine | 48±4 (-12%) | 90±6 (-5%) | 765±30 (-11%) |

Ces résultats démontrent bien que ces produits sont capables de prévenir les diminutions des taux de dopamine, d'acide dihydroxy-3,4 phénylacétique et d'acide homovanillique induites par la MPTP dans le stiatum.

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins un anticonvulsivant choisi parmi la carbamazépine et l'oxcarbazépine sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale ou parentérale.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :

| | |
|---|---|
| - Produit actif | 50 mg |
| - Mannitol | 64 mg |
| - Cellulose microcristalline | 50 mg |
| - Polyvidone excipient | 12 mg |
| - Carboxyméthylamidon sodique | 16 mg |
| - Talc | 4 mg |
| - Stéarate de magnésium | 2 mg |
| - Silice colloïdale anhydre | 2 mg |
| - Mélange de méthylhydroxypropylcellulose, polyéthylèneglycol 6000, dioxyde de titane (72-3,5-24,5) q.s.p. 1 comprimé pelliculé terminé à 245 mg | |

### Exemple B

On prépare, selon la technique habituelle, des gélules dosées à 50 mg de produit actif ayant la composition suivante :

| | |
|---|---|
| - Produit actif | 50 mg |
| - Cellulose | 18 mg |
| - Lactose | 55 mg |
| - Silice colloïdale | 1 mg |
| - Carboxyméthylamidon sodique | 10 mg |
| -Talc | 10 mg |
| - Stéarate de magnésium | 1 mg |

### Exemple C

On prépare une solution injectable contenant 10 mg de produit actif ayant la composition suivante :

| | |
|---|---|
| - Produit actif | 10 mg |
| - Acide benzoïque | 80 mg |
| - Alcool benzylique | 0,06 cm3 |
| - Benzoate de sodium | 80 mg |
| - Ethanol à 95 % | 0,4 cm3 |
| - Hydroxyde de sodium | 24 mg |
| - Propylène glycol | 1,6 cm3 |
| - Eau q.s.p. | 4 cm3 |

L'invention concerne également le procédé de préparation de médicaments utiles dans le traitement de la maladie de Parkinson et des syndromes parkinsoniens consistant à mélanger un anticonvulsivant choisi parmi la carbamazépine et l'oxcarbazépine ou les sels pharmaceutiquement acceptables de ces composés avec un ou plusieurs diluants et/ou adjuvants compatibles et pharmaceutiquement acceptables.

L'invention concerne également une méthode de traitement d'un mammifère et, notamment l'homme, présentant la maladie de Parkinson ou des syndromes parkinsoniens comprenant l'administration d'une quantité efficace de carbamazépine ou d'oxcarbazépine ou des sels pharmaceutiquement acceptables de ces composé.

## Revendications

1. Application d'un anticonvulsivant choisi parmi la carbamazépine et l'oxcarbazépine ou les sels pharmaceutiquement acceptables de ces composés à la préparation de médicaments destinés au traitement de la maladie de Parkinson et des syndromes parkinsoniens.

2. Application selon la revendication 1 pour obtenir un médicament comprenant 25 à 200 mg de carbamazépine ou d'oxcarbazépine.

## Claims

1. Use of an anticonvulsant chosen from carbamazepine and oxcarbazepine or the pharmaceutically acceptable salts of these compounds in the preparation of medicinal products intended for the treatment of Parkinson's disease and parkinsonian syndromes.

2. Use according to claim 1 for obtaining a medicinal product comprising 25 to 200 mg of carbamazepine or of oxcarbazepine.

## Patentansprüche

1. Verwendung eines Antikonvulsivums, ausgewählt aus Carbamazepin und Oxcarbazepin oder den pharmazeutisch verträglichen Salzen dieser Verbindungen, zur Herstellung von Arzneimitteln zur Behandlung der Parkinson-Krankheit und von Parkinson-Syndromen.

2. Verwendung nach Anspruch 1 zum Erhalt eines Medikaments, umfassend 25 bis 200 mg Carbamazepin oder Oxcarbazepin.
